(19) Europäisches Patentamt
European Patent Office
Office européen des brevets

(11) Veröffentlichungsnummer: **0 005 794**
**B1**

(12) # EUROPÄISCHE PATENTSCHRIFT

(45) Veröffentlichungstag der Patentschrift: **05.01.83**

(51) Int. Cl.³: **C 07 D 233/60**

(21) Anmeldenummer: **79101582.9**

(22) Anmeldetag: **22.05.79**

(54) **Verfahren zur stereospezifischen bzw. stereoselektiven Herstellung von Imidazolyl-oximetherderivaten und Imidazolyloximderivaten.**

(30) Priorität: **24.05.78 CH 5653/78**
**09.01.79 CH 161/79**

(43) Veröffentlichungstag der Anmeldung:
**12.12.79 Patentblatt 79/25**

(45) Bekanntmachung des Hinweises auf die Patenterteilung:
**05.01.83 Patentblatt 83/1**

(84) Benannte Vertragsstaaten:
**AT BE CH DE FR GB IT LU NL SE**

(56) Entgegenhaltungen:
**DE - A - 2 311 177**
**DE - A - 2 657 578**
**US - A - 4 124 767**

(73) Patentinhaber: **SIEGFRIED AKTIENGESELLSCHAFT**
**CH-4800 Zofingen (CH)**

(72) Erfinder: **Mixich, Georg, Dr.**
**Mühlemattstrasse 2**
**CH-4800 Zofingen (CH)**
Erfinder: **Thiele, Kurt, Dr.**
**Rebbergstrasse 47 d**
**CH-4800 Zofingen (CH)**

(74) Vertreter: **Jaeger, Klaus, Dr.rer.nat. Dipl.-Chem.**
**Jaeger, Grams & Pontani Patentanwälte**
**Bergstrasse 48 1/2**
**D-8035 München-Gauting (DE)**

**EP 0 005 794 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur stereospezifischen bzw. stereoselektiven Herstellung von Imidazolyl-oximetherderivaten und Imidazolyloximderivaten

Die Erfindung betrifft ein Verfahren zur stereoselektiven bzw. stereospezifischen Herstellung von Imidazol-1-yl-oximderivaten der chemischen Formeln ($II_t$) und ($II_c$):

($II_t$)

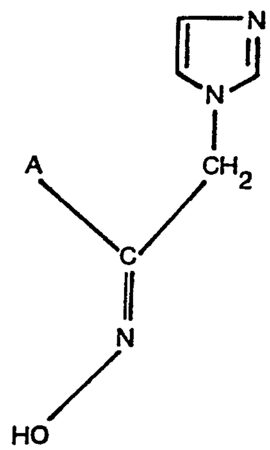

*trans*

und

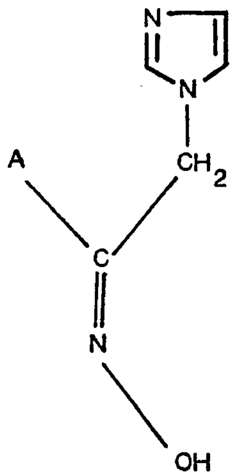

($II_c$)

*cis*

bzw. von Imidazol-1-yl-oximetherderivaten der chemischen Formeln ($I_t$) und ($I_c$) und gegebenenfalls Oximetheraddukten mit für den jeweils beabsichtigten Verwendungszweck des Produktes unbedenklichen Säuren

($I_t$)

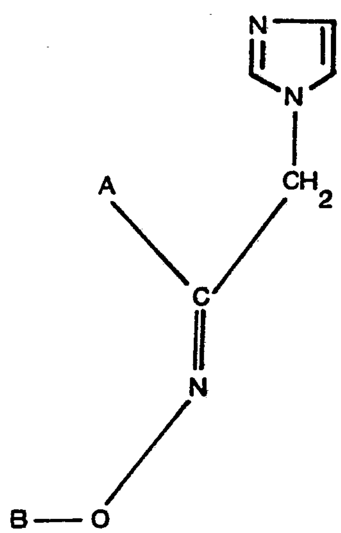

*trans*

und

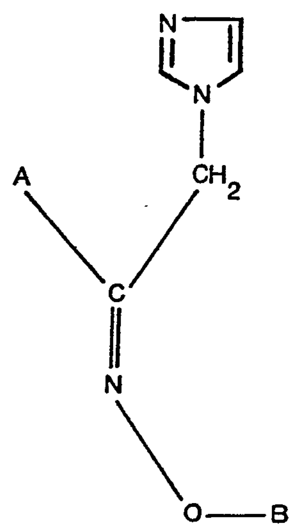

($I_c$)

*cis*

wobei

A Phenyl ist, das einfach oder zweifach mit Halogen substituiert sein kann und

B ein gesättigter aliphatischer Rest mit bis zu 8 Kohlenstoffatomen ist, der an Phenylreste gemäß der Definition für A gebunden sein kann, bedeutet

durch Umsetzung von entsprechenden 1-substituierten 2-Halogenethanonen der Formel (IV):

$$A—CO—CH_2—Hal \qquad (IV)$$

in Anwesenheit eines Solvens mit Imidazol oder einem entsprechenden Imidazolderivat und Hydroxylamin oder einem unter den Reaktionsbedingungen Hydroxylamin freisetzenden Hydroxylaminderivats zum Imidazol-1-yl-oxim der Formel (II):

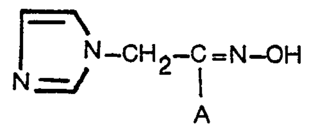

(II)

und gegebenenfalls durch entsprechende Veretherung des Metalloximats von (II) in einem polaren Solvents mit einer Halogenverbindung der Formel (III)

Hal-B (III)

sowie gegebenenfalls nachfolgende Fällung als Säureaddukt, wobei jeweils A, und B die oben genannte Bedeutung besitzen und Hal Halogen ist.

Im Rahmen der vorliegend in der Beschreibung und in den Ansprüchen dargelegten Erfindung sind die Begriffe "trans" und "cis" im Sinne der vorstehend dargestellten chemischen Formeln ($I_t$) und ($I_c$) bzw. ($II_t$) und ($II_c$) definiert. Im E/Z-System (C.A. Service, *1968*, Blackwood et al. und IUPAC Tentative Rules for the Nomenclature of Organic Chemistry) entsprechen die durch die Formel ($I_t$) bzw. ($II_t$) definierte "trans-Form" dem E-Isomer, wenn der Rest A ein bustituierter oder unsubstituierter Phenylrest ist, während die "cis-Form" der Formel ($I_c$) bzw. ($II_c$) unter diesen Bedingungen dem Z-Isomer entspricht.

Imidazolyl-oximderivate der chemischen Formel (II) und aus diesen hergestellte Imidazolyloximetherderivate sind aus der DE—OS 26 57 578 bekannt. In jener Druckschrift ist auch beschrieben, daß bei der Oximierung von 2,4-Dichlorphenacylimidazol mit Hydroxylammoniumchlorid in Ethanol in Gegenwart von Pyridin eines der beiden möglichen stereoisomeren Oxime gebildet wird, während unter gleichen Bedingungen in Abwesenheit von Pyridin das andere der beiden stereoisomeren Oxime gebildet wird. Dabei wurden relativ saubere 1-(2,4-Dichlorphenyl)-2-(1H-imidazol-1-yl)-ethanonoxime erhalten. Eine stereochemische Zuordnung der Substanzen erfolgte nicht.

Weitere Arbeiten in dieser Richtung haben inzwischen aber gezeigt, daß diese Reaktion im technischen Maßstab nicht ausreichend reproduzierbar ist und entgegen allen Erwartungen auch nicht auf analoge Reaktionen übertragbar ist. Bei den Versuchen zur analogen Übertragung und zur Durchführung der stereospezifischen Synthese im Technikumsmaßstab oder Produktionsmaßstab wurden Isomerengemische erhalten, nicht aber die reinen Isomeren.

Die in der DE—OS 26 57 578 beschriebenen 1-(2,4-Dichlorphenyl)-2-(1H-imidazol-1-yl)-ethanonoxime werden dann in der dort beschriebenen Weise in DMF in Gegenwart von Natriumhydrid bei von Raumtemperatur bis auf 80°C allmählich angehobener Reaktionstemperatur mit 2,4-Dichlorbenzylchlorid verethert. Bei dieser Veretherung sollen dann die stereochemisch ebenfalls nicht zugeordneten stereoisomeren 1-(2,4-Dichlorphenyl)-2-(1H-imidazol-1-yl)-ethanonoxim-(2,4-dichlorbenzyl)-ether erhalten werden.

Weitere Arbeiten in dieser Richtung haben inzwischen jedoch ebenfalls gezeigt, daß die bekannte Veretherung keineswegs stereospezifisch verläuft, sobalt sie in großen Ansätzen zu Produktionszwecken durchgeführt wird. Die NMR-Spektren zeigen deutlich, daß unter solchen Bedingungen stets auch erhebliche Anteile des jeweils anderen Stereoisomers im Endprodukt enthalten sind, selbst wenn bei kleinen Ansätzen nach Reinigung die sterisch reinen Isomeren erhältlich sind.

Es ist jedoch technisch durchaus wünschenswert, die wegen ihrer generell hohen antimykotischen Aktivität geschätzten wertvollen Imidazolyloximderivate und -oximetherderivate auch in Form ihrer reinen Stereoisomeren auch in größeren Ansätzen verläßlich herzustellen. Dies ist im Laboratoriumsmaßstab zwar ohne weiteres, beispielsweise auch durch chromatographische Trennung, möglich, jedoch ist eine solche chromatographische Trennung für die technische Produktion dieser Wirkstoffe ein auch aus wirtschaftlicher Sicht ungünstiges Verfahren.

Angesichts dieses Standes der Technik liegt der Erfindung die Aufgabe zugrunde ein Verfahren zur stereospezifischen Herstellung von Imidazolyl-oximetherderivaten und Imidazolyloximderivaten der eingangs genannten Formeln ($I_t$) und ($I_c$) bzw. ($II_t$) und ($II_c$) zu schaffen, das unabhängig vom Ansatzmaßstab verläßlich und reproduzierbar zu den reinen Stereoisomeren führt und mit gleichem Erfolg für alle speziellen Substanzen der in Rede stehenden Imidazolyloximetherderivate anwendbar ist.

Nach der vorliegenden Erfindung wird diese Aufgabe in der Weise gelöst, daß das Oximderivat der Formel (II) in Form jeweils eines seiner beiden Stereoisomeren

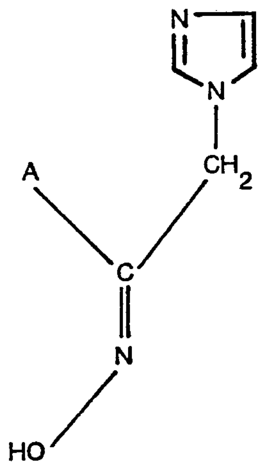

($II_t$) oder

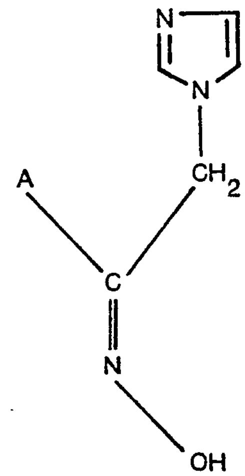

($II_c$)

*trans-Form*

*cis-Form*

bei Raumtemperatur in einem polaren organischen Lösungsmittel suspendiert oder gelöst und anschließend mit einer bei Raumtemperatur zur Metalloximatbildung befähigten Metallverbindung versetzt wird, und daß dann in der so hergestellten Lösung nach Zugabe des Halogenderivats der Formel (III) die Veretherung bei einer Temperatur von nicht höher als 40°C durchgeführt wird, wobei zur Herstellung sowohl des trans-Oxims der Formel ($II_t$) als auch des cis-Oxims der Formel ($II_c$) zunächst von einem entsprechenden 2-Halogenethanon der allgemeinen Formel IV

$$A—CO—CH_2—Hal \qquad (IV)$$

ausgegangen wird, in der A die oben genannte Bedeutung hat und Hal ein Halogenatom ist, dann aber zur Herstellung der cis-Oxime der Formel ($II_c$) das Ethanon der Formel (IV) zunächst mit Imidazol oder mit einem entsprechenden Imidazolderivat zum entsprechenden 2-(Imidazol-1-yl)-Derivat und anschließend in der Wärme in alkoholischer Lösung mit Hydroxylamin oder einem unter den Reaktionsbedingungen Hydroxylamin freisetzenden Hydroxylaminderivat zum cis-Oxim der Formel ($II_c$) umsetzt, wogegen zur Herstellung der trans-Oxime der Formel ($II_t$) das Ethanon der Formel (IV) zunächst in alkoholischer Lösung höchstens unwesentlich oberhalb Raumtemperatur mit Hydroxylamin oder einem Hydroxylamin abgebenden Hydroxylaminderivat zum 2-Halogen-cis-oxim der Formel ($V_c$)

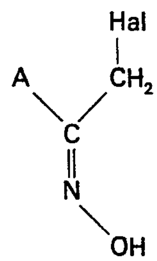

($V_c$)

umgesetzt und dann mit Imidazol oder einem entsprechenden Imidazolderivat in einem aprotischen Lösungsmittel bei 0—5°C unter cis-trans-Umlagerung zum trans-Oxim der Formel ($II_t$) umgesetzt wird.

Die stereoisomeren Imidazolyl-oximderivate der Formeln ($II_t$) und ($II_c$) die auch als solche antimykotisch und bakterizid wirken und daher wertvolle Substanzen sind, werden erfindungsgemäß in der Weise hergestellt, daß man zur Herstellung sowohl des cis-Oxims als auch des trans-Oxims von einem entsprechenden 2-Halogenethanon der allgemeinen Formel

$$A—CO—CH_2—Hal \qquad (IV)$$

in der A die oben genannte Bedeutung hat und Hal ein Halogenatom ist, ausgeht, dann aber zur Herstellung der cis-Oxime der Formel ($II_c$) das Ethanon der Formel (IV) zunächst mit Imidazol oder einem entsprechenden Imidazolderivat zum entsprechenden 2-(Imidazol-1-yl)-Derivat und anschließend in der Wärme in alkoholischer Lösung mit Hydroxylamin oder einem unter den Reaktionsbedingungen Hydroxylamin freisetzenden Hydroxylaminderivat zum cis-Oxim der Formel ($II_c$) umsetzt, wogegen zur Herstellung der trans-Oxime der Formel ($II_t$) das Ethanon der Formel (IV) zunächst in alkoholischer Lösung höchstens unwesentlich oberhalb Raumtemperatur mit Hydroxylamin oder einem Hydroxylamin abgebenden Hydroxylaminderivat zum 2-Halogen-cis-oxim der Formel ($V_c$)

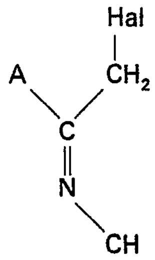

(V$_c$)

umgesetzt und dann mit Imidazol oder einem entsprechenden Imidazolderivat in einem aprotischen Lösungsmittel bei 0.5°C unter cis-trans-Umlagerung zum trans-Oxim der Formel (II$_t$) umgesetzt wird. Diese unter cis-trans-Umlagerung erfolgende Imidazolierung des 2-Halogen-cis-oxims der Formel (V$_c$) wird vorzugsweise in einem unpolaren aprotischen Lösungsmittel, beispielsweise Chloroform, durchgeführt.

In dem einen einzigen bekannten und einleitend erörterten Fall ist bislang eine mehr oder minder stereoselektive Herstellung der stereoisomeren Imidazolyl-oximetherderivate der Formeln (I$_t$) und (I$_c$) durch eine im kleinen Ansatz im wesentlichen stereospezifische Veretherung in nicht ausreichend reporduzierbarer und nicht analog übertragbarer Weise gelungen. Demgegenüber schafft die Erfindung ein sicher und für alle Substanzen der in Rede stehenden Klasse der Oximether reporduzierbares und auch im Produktionsmaßstab durchführbares Verfahren zur stereoselektiven und stereospezifischen Herstellung der cis-trans-isomeren oder E/Z-isomeren Imidazolyl-oximetherderivate.

Das gleiche gilt für die als Zwischenprodukte benötigten, aber auch selbst wertvolle Wirkstoffe darstellenden Imidazolyl-ethanonoximderivate in ihren stereoisomeren Formen der Formeln (II$_t$) und (II$_c$). Nur in dem vorstehend diskutierten Fall ist bislang eine stereoselektive Herstellung der Oxime der allgemeinen Formeln (II$_t$) und (II$_c$) in nicht ausreichend reproduzierbarer und nicht analog übertragbarer Weise gelungen. Demgegenüber schafft die Erfindung weiterhin ein sicher reproduzierbares und auch im technischen Maßstab durchführbares Verfahren zur stereoselektiven Herstellung stereoisomerer Oxime. Dabei wird zur Herstellung des cis-Oxims der allgemeinen Formel (II$_c$) das entsprechende 2-Halogenethanon der Formel (IV) zunächst imidazoliert und erst anschließend oximiert, während umgekehrt zur Herstellung des trans-Oxims der allgemeinen Formel (II$_t$) das 2-Halogenethanon zuerst oximiert wird, und zwar zum 2-Halogen-*cis*-oxim der Formel (V$_c$), welches sich dann bei nachfolgender Imidazolierung in die trans-Form umwandelt (vgl. J. Amer. Chem. Soc. *94*, 9274—77).

Die stereoisomeren Oxime der allgemeinen Formeln (II$_t$) und (II$_c$) zeigen ein unterschiedliches chromatographisches Verhalten, insbesondere im Dünnschichtchromatogramm deutlich verschiedene RF-Werte, und können aufgrund ihrer NMR-spektroskopischen Daten zweifelsfrei jeweils einer der beiden stereoisomeren Formen zugeordnet werden.

Auch die stereoisomeren Imidazolyl-oximetherderivate der Formeln (I$_t$) und (I$_c$) zeigen ein deutlich unterschiedliches chromatographisches Verhalten, ebenfalls insbesondere im Dünnschichtchromatogramm deutlich verschiedene RF-Werte. Eine Zuordnung der Produkte jeweils zur cis-Form oder trans-Form bzw. zur (E)-Form oder (Z)-Form kann aus den NMR-Spektren eindeutig vorgenommen werden. Diese Zuordnung ist ebenfalls insbesondere aus der Verschiebung der $CH_2$-Protonen möglich ($\delta_{cis} > \delta_{trans}$).

Nach einer Weiterbildung der Erfindung wird zur Herstellung der cis-Isomeren der Formel (I$_c$) die stereospezifische Veretherung vorzugsweise in Aceton in Verbindung mit KOH durchgeführt, insbesondere in Gegenwart von geringfügig weniger, vorzugsweise 5 bis 15 Mol-% weniger, als der äquimolaren Menge KOH entspricht, während zur Herstellung der trans-Isomeren der Formel (I$_t$) vorzugsweise in Dimethylformamid (DMF) in Verbindung mit NaH gearbeitet wird. In beiden Fällen werden die so hergestellten stereoisomeren Imidazolyl-oximetherderivate vorzugsweise durch Fällen als Nitrate mit wässriger Salpetersäure aus dem Reaktionsgemisch isoliert. Die Fällung kann aber auch mit anderen teleologisch unbendenklichen anorganischen und organischen Säuren erfolgen. Die als Zwischenprodukt benötigten stereoisomer reinen Imidazolyl-ethanonoximderivate der Formel (II$_t$) und (II$_c$) können in der vorstehend beschriebenen Weise dargestellt werden, sind aber auch durch säulenchromatographische oder andere Isomerentrennung zugänglich.

Das Verfahren der Erfindung zeichnet sich vor allem durch seine Zuverlässigkeit und seine Anwendbarkeit auf die gesamte durch die chemischen Formeln I bzw. II definierte Substanzklasse aus. Von besonderem Interesse sind dabei jedoch im Rahmen der durch die chemischen Formeln I und II definierten Substanzklasse jene Substanzen, bei denen in den Formeln I und II bzw. I bis V der Rest A ein unsubstituierter oder ein durch Halogen, speziell Chlor, einfach oder zweifach substituierter Phenylrest, der Rest B ein unsubstituierter oder ein im Benzolring einfach oder zweifach durch Halogen speziell Chlor substituierter, substituierter Phenylalkylrest mit 1 bis 4 Kohlenstoffatomen im Alkylteil, speziell Benzylrest, ist. Die größte Bedeutung des Verfahrens der Erfindung liegt darin, jene Substanzen auch im produktionstechnischen Maßstab in stereochemisch reiner Form zugänglich werden zu lassen, für die in den Formeln I und II der Rest A der 4-Chlorphenyl- oder der 2,4-Dichlorphenylrest und der Rest B der 4-Chlorbenzyl- oder der 2,4-Dichlorbenzylrest sind.

Die Substanzen der Formeln I und II sind fungizid und bakterizid und finden sowohl in der Humanmedizin und in der Veterinärmedizin als auch im Pflanzenschutzbereich als Wirkstoffe in entsprechend formulierten Präparaten Verwendung.

Die Erfindung ist im folgenden anhand von Ausführungsbeispielen näher erläutert.

Beispiel 1

(Z)-1-(2,4-Dichlorphenyl)-2-(1H-imidazol-1-yl)-O-(2,4-dichlorbenzyl)-ethanonoximnitrat

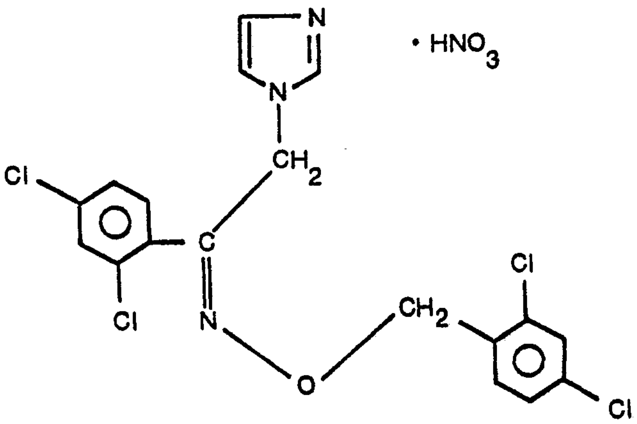

2064 g (7,64 mol) (Z)-1-(2,4-Dichlorphenyl)-2-(1H-imidazol-1-yl)-ethanonoxim mit einem Schmelzpunkt von 216 bis 218°C werden in der fünffachen Menge (10, 3 l) Aceton suspendiert, kräftig gerührt und mit 450 g KOH (90%-ig) in fein pulvrisierter Form versetzt. Dabei wird das suspendierte Ethanonoxim allmählich gelöst. Nach ungefähr 1 h wird die Lösung mit 1554 g (7,9 mol) 2,4-Dichlorbenzylchlorid in einer Portion versetzt. Die dabei eintretende Reaktion is schwach exotherm und erwärmt das Reaktionsgemisch auf 35 bis höchstens 40°C. Bis zum Abschluß der Reaktion wird noch 4 h gerührt, wobei erforderlichenfalls durch gelinde Kühlung darauf geachtet wird, daß die Temperatur im Reaktionsgemisch nicht größer als 40°C wird. Nach dem Abnschluß der Reaktion wird das Reaktionsgemisch auf 15 1 Wasser gegossen.

Anschließend wird das Reaktionsprodukt mit 6 l wässriger 2n $HNO_3$ als Nitrat gefällt. Einige Stunden nach dem Fällen des Nitrats wird abgesaugt und gut mit Wasser ausgewaschen. Das so abgetrennte rohe Nitrat wird dann aus 11,3 l 94%-igem Ethanol umkristallisiert. Eine zweite Umkristallisation aus der dreifachen Menge Ethanol liefert dann das Endprodukt mit einer Reinheit von 99,95 Form farbloser Kristalle. Die Reinheit des so erhaltenen Produktes wird durch die Thermoanalyse überwacht. Der Schmelzpunkt der erhaltenen Kristalle liegt bei 139,5 bis 140,5°C. Die Ausbeute beträgt 62,5%.

Elementaranalyse für $C_{18}H_{13}Cl_4N_3O \cdot HNO_3$ (MG 492,17)

| | C(%) | H(%) | N(%) | Cl(%) |
|---|---|---|---|---|
| berechnet | 43,93 | 2,87 | 11,38 | 28,82 |
| gefunden | 43,61 | 2,88 | 11,03 | 28,90 |

Das NMR-Spektrum zeigt für die $CH_2$-Protonenverschiebung $\delta$-Werte von 5,34 und 5,64 ppm gegenüber 5,08 und 5,35 ppm für das andere Isomere. Dieser Befund ermöglicht die Zuordnung des erhaltenen Produkts zur (Z)-Form, da für diese die größeren Protonenverschiebungen zu erwarten sind.

In gleicher Weise wird das (Z)-1-(2,4-Dichlorphenyl)-2-(1H-imidazol-1-yl)-O-benzyl-ethanonoximnitrat (F.130—132°C) hergestellt, wobei in dem vorstehend beschriebenen Verfahren lediglich statt des 2,4-Dichlorbenzylchlorids die äquimolare Menge Benzylchlorid eingesetzt wird.

Beispiel 2

(E)-1-(2,4-Dichlorphenyl)-2-(1H-imidazol-1-yl)-O-(2,4-dichlorbenzyl)-ethanonoximnitrat

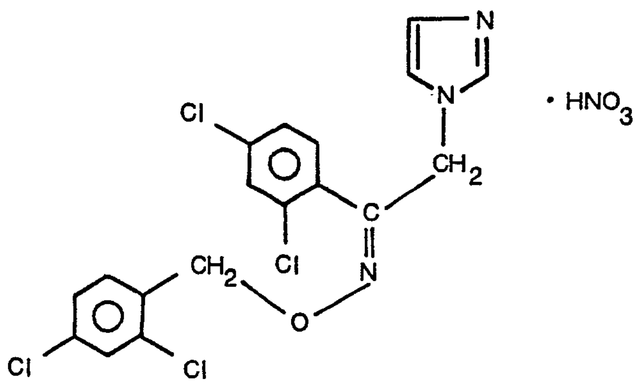

27 g (0,1 mol) (E)-1-(2,4-Dichlorphenyl)-2-(1H-imidazol-1-yl)-ethanonoxim werden in 100 ml DMF gelöst. Die Lösung wird mit 2,4 g (0,1 mol) NaH versetzt und 1 h bei Raumtemperatur gerührt. Anschließend werden unter Rühren 20 g (>0,1 mol) 2,4-Dichlorbenzylchlorid in 100 ml DMF zugetropft. Nach 1 h wird das Lösungsmittel am Rotationsverdampfer abgezogen. Der Rückstand wird mit Wasser verrührt und mit wässriger 2n $HNO_3$ zur Kistallisation gebracht. Das so erhaltene rohe Nitrat wird nach dem Absaugen und Trocknen aus Alkohol umkristallisiert. Dabei werden 10,5 g gereinigtes Endprodukt in Form farbloser Kristalle vom Schmelzpunkt 164 bis 165°C erhalten.

Elementaranalyse für $C_{18}H_{14}Cl_4N_3O \cdot HNO_3$ (MG 492,17)

| | C(%) | H(%) | N(%) | Cl(%) |
|---|---|---|---|---|
| berechnet | 43,93 | 2,87 | 11,38 | 28,82 |
| gefunden | 43,82 | 2,79 | 10,99 | 28,90 |

Im NMR-Spektrum werden für die $CH_2$-Protonen $\delta$-Werte von 5,08 und 5,35 ppm beobachtet. Diese Werte sind kleiner als die für das andere Isomere gefundenen Verschiebungswerte und erlauben daher die Zuordnung des nach diesem Beispiel 2 hergestellten Isomers zur (E)-Form.

Beispiel 3

(E)-1-(4-Chlorphenyl)-2-(1H-imidazol-1-yl)-O-(4-chlorbenzyl)-ethanononoximnitrat

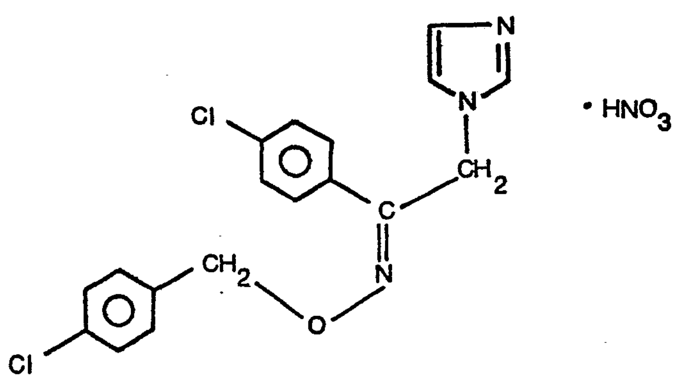

Das im Beispiel 2 beschriebene Verfahren wird mit der Abänderung wiederholt, daß statt des 2,4-Dichlorbenzylchlorids die äquivalente Menge p-Chlorbenzylchlorid eingesetzt wird.

Das farblos kristalline Reaktionsprodukt hat nach dem Umkristallisieren aus Ethanol einen Schmelzpunkt von 172°C.

Beispiel 4

(Z)-1-(4-Chlorphenyl)-2-(1H-imidazol-1-yl)-O-(4-chlorbenzyl)-ethanonoximnitrat

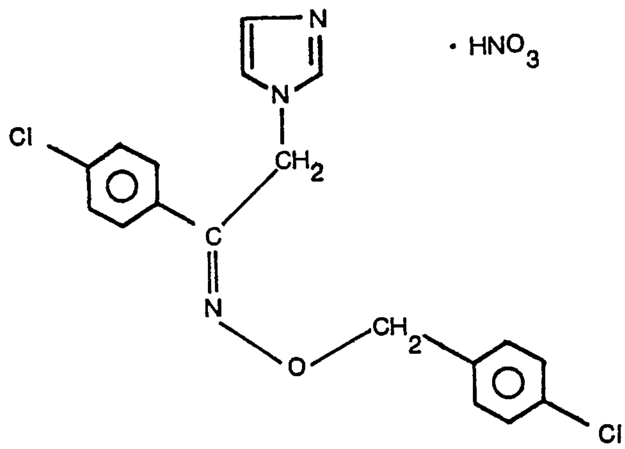

Das im Beispiel 1 beschriebene Verfahren wird mit der Abänderung wiederholt, daß statt des 2,4-Dichlorbenzylchlorids die äquivalente Menge p-Chlorbenzylchlorid eingesetzt wird.

Nach dem Fällen mit wässriger Salpetersäure und Umkristallisieren aus Ethanol wird das reine Endprodukt in Form farbloser Kristalle mit einem Schmelzpunkt von 161°C erhalten.

Beispiel 5

E-1-(4-Chlorphenyl)-2-(1H-imidazol-1-yl)-ethanonoxim

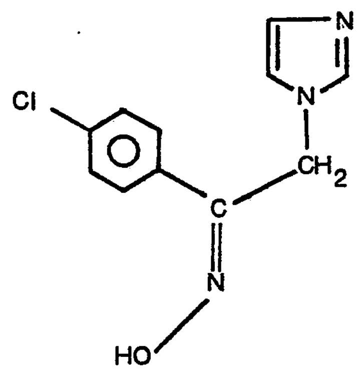

23,4 g (0,1 mol) p-Chlorphenacylbromid werden in 300 ml Methanol gelöst und mit 20,9 g (0,3 mol) Hydroxylammoniumchlorid versetzt. Anschließend wird kurz gerührt und ca. 14 bis 16 h bei Raumtemperatur stehengelassen. Dann wird mit Wasser verdünnt, wobei das Reaktionsprodukt ausfällt. Der Niederschlag wird abgesaugt, mit Wasser gewaschen und getrocknet. Es werden 20 g bereits recht reines Rohprodukt erhalten, die anschließend aus Benzin umkristallisiert werden. Das erhaltene Z-1-(4-Chlorphenyl)-2-bromethanonoxim bildet farblose Kristalle mit einem Schmelzpunkt von 106°C.

5 g (0,02 mol) des erhaltenen Halogen-Z-Oxims werden in kleinen Portionen in eine vorgelegte Lösung von 4 g (0,06 mol) Imidazol in 40 ml Chloroform gegeben. Dabei wird durch ein Eisbad eine Reaktionstemperatur im Bereich zwischen 0 und 5°C eingehalten. Das Eintragen des Halogen-Oxims in die Imidazollösung erfolgt unter ständigem Rühren. Nach Abschluß der Zugabe wird weitere 3 h gerührt. Der Niederschlag wird dann abgesaugt und mit Wasser gewaschen. Es werden 3 g Rohprodukt erhalten, die aus Alkohol umkristallisiert werden. Das so hergestellte stereochemisch reine E-1-(4-Chlorphenyl)-2-(1H-imidazol-1-yl)-ethanonoxim bildet farblose Kristalle mit einen Schmelzpunkt von 185 bis 186°C.

Elementaranalyse für $C_{11}H_{10}ClN_3O$ (235,7)

| | C(%) | H(%) | N(%) | Cl(%) |
|---|---|---|---|---|
| berechnet | 56,06 | 4,28 | 17,83 | 15,04 |
| gefunden | 56,21 | 4,30 | 17,22 | 15,18 |

Beispiel 6

Z-1-(4-Chlorphenyl)-2-(1H-imidazol-1-yl)-ethanonoxim

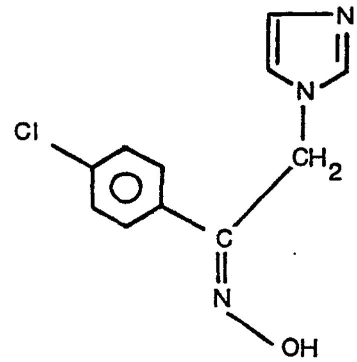

89,2 g 4-Chlorphenacylbromid werden in 170 ml Chloroform (oder Methylenchlorid) gelöst und zu einer Lösung von 156 g Imidazol in 600 ml Chloroform (oder Methylenchlorid) bei 0 bis 5°C zugetropft. Danach wird während 4 h bei dieser Temperatur weitergerührt, das Lösungsmittel im Rotationsverdampfer abdestilliert und der Rückstand mit Wasser behandelt. Nach dem Absaugen und Trocknen erhält man aus Toluol 65 g p-Chlorphenacyl-imidazol oder 1-(4-Chlorphenyl)-2-(1H-imidazol-1-yl)-1-ethanon als farblose Kristalle vom Schmelzpunkt 158 bis 160°C.

11 g (0,05 mol) des so erhaltenen p-Chlorphenacyls werden zusammen mit 5,2 g (0,075 mol) Hydroxylammoniumchlorid in 110 ml Ethanol gelöst. Unter Rühren werden in diese Lösung 10 g (0,25 mol) festes Natriumhydroxid gegeben. Anschließend wird 1 h unter Rückfluß auf Siedetemperatur erwärmt. Nach dem Abkühlen wird das Reaktionsgemisch in 175 ml wässrige 1n HCl gegossen, wobei das Oxim ausfällt. Die auf diese Weise in fast quantitativer Ausbeute erhaltene Rohsubstanz wird aus Alkohol umkristallisiert. Dabei wird das Z-1-(4-Chlorphenyl)-2-(1H-imidazol-1-yl)-ethanonoxim in Form farbloser Kristalle vom Schmelzpunkt 194 bis 195°C erhalten.

Elementaranalyse für $C_{11}H_{10}ClN_3O$ (235,7)

| | C(%) | H(%) | N(%) | Cl(%) |
|---|---|---|---|---|
| berechnet | 56,06 | 4,28 | 17,83 | 15,04 |
| gefunden | 56,22 | 4,31 | 17,45 | 15,12 |

In der im Beispiel 5 beschriebenen Weise wird im weiteren die folgende Verbindung hergestellt: E-1-(2,4-Dichlorphenyl)-2-(1H-imidazol-1-yl)-ethanonoxim (F.205—206°C).

In der im Beispiel 6 beschriebenen Weise wird außerdem die folgende Verbindung hergestellt: Z-1-(2,4-Dichlorphenyl)-2-(1H-imidazol-1-yl)-ethanonoxim (F.218—220°C).

**Patentansprüche**

1. Verfahren zur stereoselektiven bzw. stereospezifischen Herstellung von Imidazol-1-yl-oximderivaten der chemischen Formeln ($II_t$) and ($II_c$):

($II_t$)

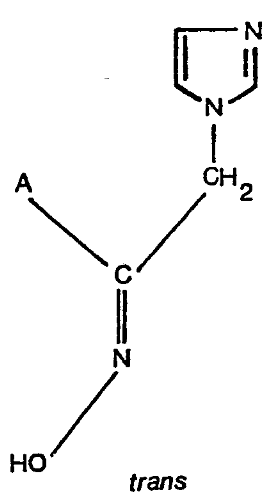

*trans*

und

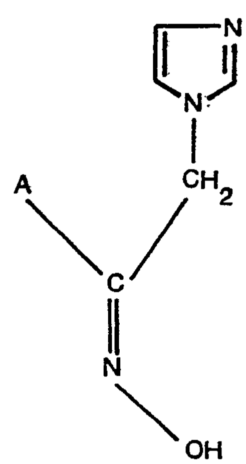

($II_c$)

*cis*

bzw. von Imidazol-1-yl-oximetherderivaten der chemischen Formeln ($I_t$) und ($I_c$) und gegebenenfalls Oximetheraddukten mit für den jeweils beabsichtigen Verwendungszweck des Produktes unbedenklichen Säuren

($I_t$)

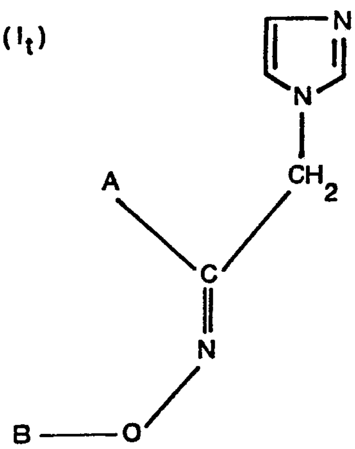

*trans*

und

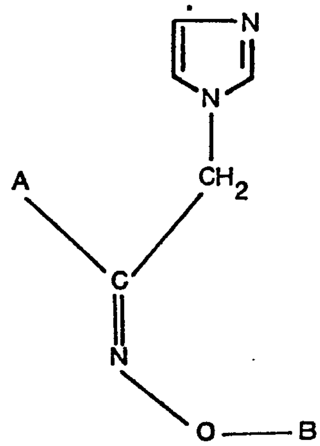

($I_c$)

*cis*

wobei
A Phenyl ist, das einfach oder zweifach mit Halogen substituiert sein kann und
B ein gesättigter aliphatischer Rest mit bis zu 8 Kohlenstoffatomen ist, der an Phenylreste gemäß der Definition für A gebunden sein kann, bedeutet.
durch Umsetzung von entsprechenden 1 substituierten 2-Halogenethanonen der Formel (IV):

$$A—CO—CH_2—Hal \qquad (IV)$$

in Anwesenheit eines Solvens mit Imidazol oder einem entsprechenden Imidazolderivat, und Hydroxylamin oder einem unter den Reaktionsbedingungen Hydroxylamin freisetzenden Hydroxylaminderivat zum Imidazol-1-yl-oxim der Formel (II):

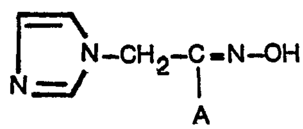

(II)

und gegebenenfalls durch entsprechende Veretherung des Metalloximats von (II) in einem polaren Solvens mit einer Halogenverbindung der Formel (III)

$$Hal—B \qquad (III)$$

sowie gegebenenfalls nachfolgende Fällung als Säureaddukt, wobei jeweils A, und B die oben genannte Bedeutung besitzen und Hal Halogen ist, dadurch gekennzeichnet, daß zur Herstellung der *cis-Oxime* ($II_c$) das Ethanon der Formel (IV) zunächst mit Imidazol oder mit einem entsprechenden Imidazolderivat zum entsprechenden 2-(Imidazol-1-yl)-Derivat und anschließend in der Wärme in alkoholischer Lösung mit Hydroxylamin oder einem unter den Reaktionsbedingungen Hydroxylamin freisetzenden Hydroxylaminderivat umgesetzt wird, wogegen zur Herstellung der *trans-Oxime* ($II_t$) das Ethanon der Formel (IV) zunächst in alkoholischer Lösung höchstens unwesentlich oberhalb Raumtemperatur mit Hydroxylamin oder einem Hydroxylamin abgebenden Hydroxylaminderivat zum 2-Halogen-cis-oxim der Formel ($V_c$)

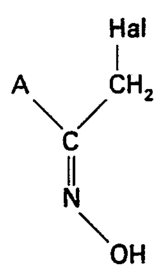

($V_c$)

umgesetzt und dann mit Imidazol oder einem entsprechenden Imidazolderivat in einem aprotischen Lösungsmittel bei 0 bis 5°C unter cis-trans-Umlagerung zum trans-Oxim der Formel ($II_t$) umgesetzt

wird und daß zur Herstellung der *cis*- und *trans-Oximether* der Formeln ($I_c$) und ($I_t$) das entsprechende stereoisomere Oxim der Formel ($II_c$) bzw. ($II_t$) bei Raumtemperatur in das Metalloximat überführt und die Veretherung bei einer Temperatur von nicht höher als 40°C durchgeführt wird.

2. Verfahren nach Anspruch 1, dadurch gekennzeichnet, daß im Falle der Herstellung der cis-Isomeren der Formel ($I_c$) das Metalloximat unter Verwendung von KOH und im Falle der Herstellung der trans-Isomeren der Formel ($I_t$) des entsprechende Metalloximat mit, bildet wobei im ersten Fall KOH vorzugsweise in einer Menge zugesetzt wird, die um 5 bis 15 Mol-% kleiner als die äquimolare Menge ist, jeweils bezogen auf das Oximderivat der Formel ($II_c$).

3. Verfahren nach einem der Ansprüche 1 oder 2, dadurch gekennzeichnet, daß die Veretherung in Aceton oder Dimethylformamid (DMF) als Lösungsmittel durchgeführt wird, wobei vorzugsweise als Lösungsmittel zur Herstellung der cis-Isomeren der Formel ($I_c$) Aceton und zur Herstellung der trans-Isomeren der Formel ($I_t$) DMF verwendet werden.

4. Verfahren entsprechend den Ansprüchen 1, 2 und 3 zur Herstellung von (Z)-1-(2,4-Dichlorphenyl)-2-(1H-imidazol-1-yl)-O-(2,4-dichlorbenzyl)-ethanonoxim (cis-Isomer) der Formel

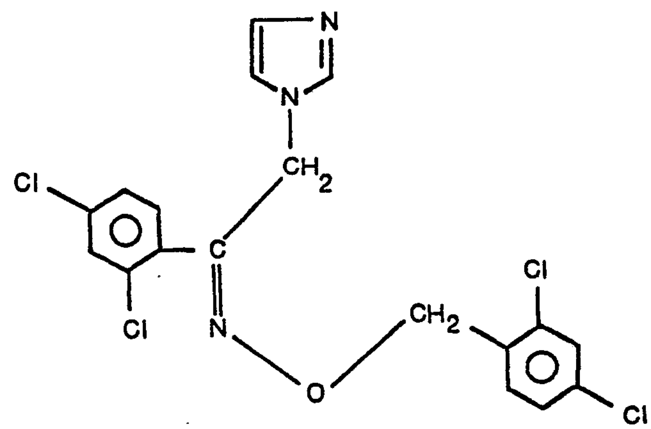

und dessen Nitrat der Formel:

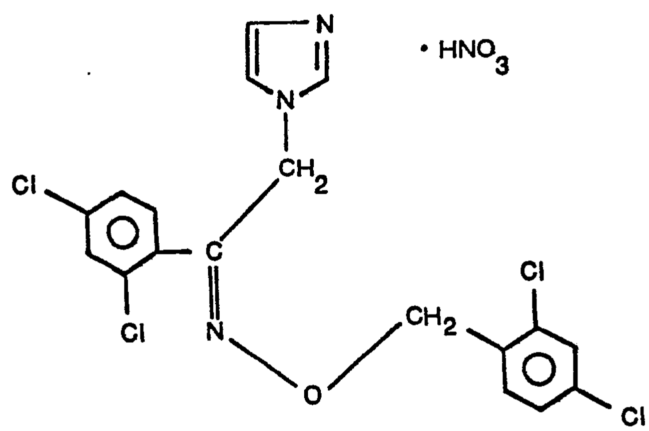

dadurch gekennzeichnet, daß (Z)-1-(2,4-Dichlorphenyl)-2-(1H-imidazol-1-yl)-ethanonoxim in der ungefähr fünffachen Menge Aceton, bezogen auf das Gewicht des Oxims, suspendiert und unter kräftigem Rühren mit festem, fein pulvrisiertem KOH versetzt wird, und zwar mit höchstens der äquimolaren Menge, vorzugsweise einer bis zu 15 Mol-% kleineren Menge, wobei so lange gerührt wird, bis das Oxim vollständig gelöst ist, daß die Lösung nach ungefähr 1 h in einer Portion mit ungefähr der äquimolaren Menge 2,4-Dichlorbenzylchlorid versetzt und anschließend ungefähr noch 4 h gerührt wird, wobei, erforderlichenfalls durch zusätzliche Kühlung, darauf geachtet wird, daß die Temperatur im Reaktionsgemisch nicht größer als 40°C wird, und daß das Reaktionsgemisch dann auf Wasser gegossen und in üblicher Weise zum Oximether aufgearbeitet wird, oder daß das wässrige Reaktionsgemisch zur Fällung und Isolierung des Nitrats mit wässriger 2n $HNO_3$ versetzt und das ausgefällte Nitrat abfiltriert und gegebenenfalls aus Ethanol umkristallisiert wird.

5. Verfahren nach Anspruch 1, dadurch gekennzeichnet, daß bei den Oximierungen als Hydroxylamin freisetzendes Reagens Hydroxylammoniumchlorid verwendet wird.

6. Verfahren nach einem der Ansprüche 1 oder 5, dadurch gekennzeichnet, daß die Oximierung des 2-Imidazol-1-yl-Derivates zum cis-Oxim der Formel ($II_c$) in einem Alkanol mit 1 oder 2 C-Atomen, insbesondere in Methanol, vorzugsweise in Gegenwart eines Alkalimetallhydroxids, bei Rückflußtemperatur durchgeführt wird.

7. Verfahren nach einem der Ansprüche 1 oder 5, dadurch gekennzeichnet, daß die Oximierung des 2-Halogenethanons der Formel (IV) zum 2-Halogen-cis-oxim der Formel ($V_c$) mit Methanol als Lösungsmittel bei Raumtemperatur erfolgt.

8. Verfahren nach einem der Ansprüche 1, 5 oder 7, dadurch gekennzeichnet, daß die unter cis-trans-Umlagerung erfolgende Imidazolierung des 2-Halogen-cis-oxims der Formel ($V_c$) in Chloroform, durchgeführt wird.

9. Verfahren nach Anspruch 1 zur Herstellung von (Z)-1-(2,4-Dichlorphenyl)-2-(1H-imidazol-1-yl)-O-(2,4-dichlorbenzyl)-ethanonoxim und dessen Säureaddukten mit für den jeweils beabsichtigten Verwendungszweck des Produktes unbedenklichen Säuren, dadurch gekennzeichnet, daß Z-1-(2,4-Dichlorphenyl)-2-(1H-imidazol-1-yl)-ethanonoxim und 2,4-Dichlorbenzylchlorid als Ausgangssubstanzen eingesetzt werden und der erhaltene Ether gegebenenfalls nachfolgend als Säureaddukt gefällt wird.

**Revendications**

1. Procédé de préparation stéréosélective ou stéréospécifique de dérivés d'imidazolyl-1-oximes répondant aux formules chimiques ($II_t$) et ($II_c$)

($II_t$)

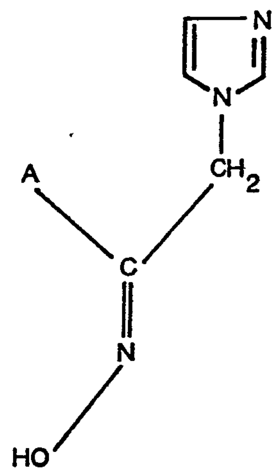

trans

et

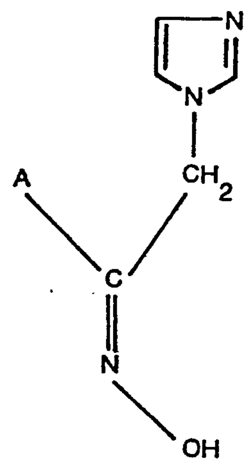

($II_c$)

cis

ou de dérivés d'éthers d'imidazolyl-1-oximes répondant aux formules chimiques ($I_t$) et ($I_c$) et éventuellement de produits d'addition, sur les éthers d'oximes, d'acides dépourvus d'inconvénient pour l'application envisagée du produit:

($I_t$)

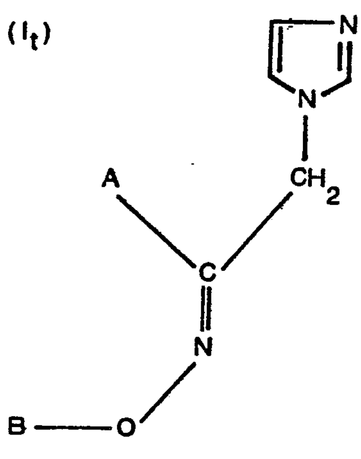

trans

et

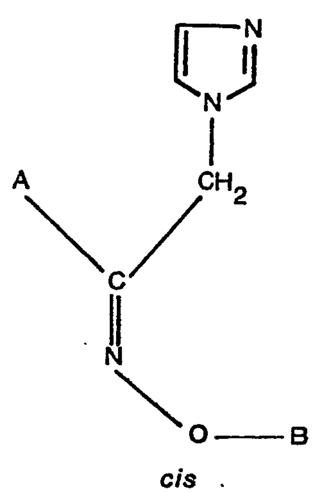

($I_c$)

cis .

formules dans lesquelles:

A est un radical phényle, qui peut être substitué une ou deux fois par de l'halogène, et

B représente un radical aliphatique sauré comportant jusqu'à 8 atomes de carbone, qui peut être relié à un radical phényle répondant à la définition donnée pour A,

par réaction d'halogéno-2 éthanones correspondantes substitutées en position 1, de formule (IV):

$$A—CO—CH_2—Hal \qquad (IV)$$

en présence d'un solvant avec l'imidazole ou un dérivé correspondant de l'imidazole, et avec l'hydroxylamine ou un dérivé d'hydroxylamine libérant de l'hydroxylamine dans les conditions de la réaction, pour obtenir l'imidazolyl-1-oxime de formule (II)

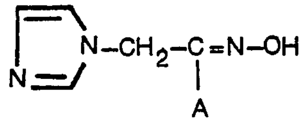

(II)

et éventuellement par éthérification correspondante du dérivé métallique de l'oxime (II) dans un solvant polaire avec un composé halogéné de formule (III)

$$Hal—B \qquad (III)$$

et éventuellement précipitation subséquente sous forme d'un produit d'addition d'acide (A et B possédant à chaque fois le sens indiqué ci-dessus et Hal étant un halogène), procédé caractérisé en ce que, pour préparer les oximes cis ($II_c$), on fait réagir l'éthanone de formule (IV) tout d'abord avec l'imidazole ou avec un dérivé d'imidazole correspondant pour obtenir le dérivé (imidazolyl 1)-2 correspondant, puis, à chaud en solution alcoolique avec l'hydroxylamine ou avec un dérivé d'hydroxylamine libérant de l'hydroxylamine dans les conditions de la réaction, alors que, pour préparer les oximes trans ($II_t$), on fait réagir l'éthanone de formule (IV) tout d'abord en solution alcoolique, à une température ne dépassant au maximum que de très peu la température ambiante, avec l'hydroxylamine ou avec un dérivé d'hydroxylamine libérant de l'hydroxylamine, pour obtenir l'halogéno-2-oxime cis de formule ($V_c$)

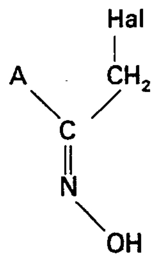

($V_c$)

et l'on fait ensuite réagir avec l'imidazole ou avec un dérivé correspondant de l'imidazole, dans un solvant approtique, entre 0 et 5°C avec transposition cis-trans, pour obtenir l'oxime trans de formule ($II_t$), et en ce que, pour produire les éthers d'oximes cis et trans répondant aux formules ($I_c$) et ($I_t$), on transforme à la température ambiante l'oxime stéréoisomère correspondante de formule ($II_c$) ou ($II_t$) en le dérivé métallique d'oxime et l'on effectue l'éthérification à une température non supérieure à 40°C.

2. Procédé selon la revendication 1, caractérisé en ce que, dans le cas de la préparation des isomères cis de formule ($I_c$), on forme le dérivé métallique de l'oxime en utilisant KOH et, dans le cas de la préparation des isomères trans de formule ($I_t$), on forme le dérivé métallique correspondant d'oxime avec NaH, et, dans le premier cas, on ajoute avantageusement KOH en une quantité qui est inférieure de 5 à 15 moles % à la quantité équimolaire, les quantités étant chaque fois rapportées au dérivé d'oxime de formule ($II_c$).

3. Procédé selon l'une des revendications 1 et 2, caractérisé en ce qu'on effectue l'éthérification dans de l'acétone ou du diméthylformamide (DMF) comme solvant, en utilisant de préférence l'acétone comme solvant pour préparer les isomères cis de formule ($I_c$) et DMF pour préparer les isomères trans de formule ($I_t$).

4. Procédé selon les revendications 1, 2 et 3, pour préparer la (Z)-(dichloro-2,4 phényl)-1 (*1H*-imidazolyl-1)-2 O-(dichloro-2,4 benzyl)-éthanone-oxime (isomère-cis) de formule

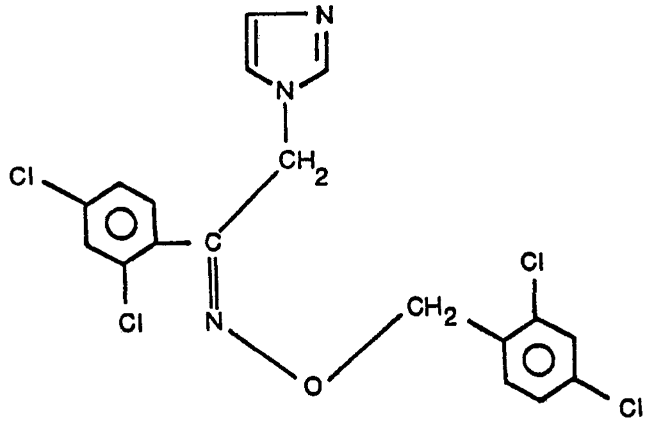

et son nitrate de formule:

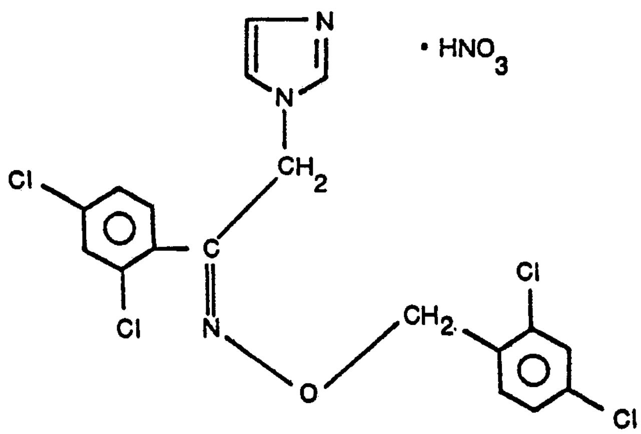

procédé caractérisé en ce que l'on met la (Z)-(dichloro-2,4 phényl)-1 (*1H*-imidazolyl-1)-2 éthanone-oxime en suspension dans une quantité environ quintuple d'acétone, par rapport au poids de l'oxime, et l'on ajoute sous agitation puissante KOH solide finement pulvérisé, cela en une quantité au plus égale à la quantité équivalente et de préférence en une quantité inférieure d'au maximum 15 moles%, en agitant jusqu'à ce que l'oxime soit totalement dissoute, au bout d'une heure environ on ajoute à la solution, en une seule fois, la quantité approximativement équimolaire de chlorure de dichloro-2,4 benzyle puis l'on agite encore durant 4 heures environ et, si nécessaire par un refroidissement supplémentaire, on veille à ce que la température n'excède pas 40°C dans le mélange réactionnel; on verse ensuite le mélange réactionnel dans de l'eau et on le soumet à un traitement complémentaire usuel conduisant à l'éther d'oxime, ou bien, pour précipiter et isoler le nitrate, on ajoute une solution aqueuse de $HNO_3$ 2N au mélange réactionnel aqueux, on filtre le nitrate précipité et on le recristallise éventuellement dans de l'éthanol.

5. Procédé selon la revendication 1, caractérisé en ce que, lors de la réaction de formation d'oxime, on utilise comme corps libérant de l'hydroxylamine le chlorure d'hydroxylammonium.

6. Procédé selon l'une des revendications 1 ou 5, caractérisé en ce qu'on effectue la transformation du dérivé (imidazolyl-1)-2 en l'oxime cis de formule ($II_c$) dans un alcanol comportant un ou deux atomes de carbone, notamment dans du méthanol, avantageusement en présence d'un hydroxyde de métal alcalin, en opérant à la température du reflux.

7. Procédé selon l'une des revendications 1 ou 5, caractérisé en ce qu'on effectue à la température ambiante la transformation de l'halogéno-2 éthanone de formule (IV) en l'halogéno-2 oxime cis de formule ($V_c$) avec du méthanol comme solvant.

8. Procédé selon l'une quelconque des revendications 1, 5 et 7, caractérisé en ce qu'on effectue dans du chloroforme la fixation, se produisant avec transposition cis-trans, de l'imidazole sur l'halogéno-2-oxime cis de formule ($V_c$).

9. Procédé selon la revendication 1 pour préparer de la (Z)-(dichloro-2,4 phényl)-1 (*1H*-imidazolyl-1)-2 O-dichloro-2,4 benzyl)-éthanone-oxime et ses composés d'addition d'acides obtenus avec des acides sans inconvénient pour l'application à laquelle on destine le produit, procédé caractérisé en ce que l'on utilise comme substances de départ la Z-(dichloro-2,4 phényl)-1 (*1H*-imidazolyl-1)-2 éthanone-oxime et le chlorure de dichloro-2,4 benzyle, et en ce qu'on précipite éventuellement ensuite, comme produit d'addition d'acide, l'éther obtenu.

## Claims

1 A method for stereoselectively and, respectively, stereospecifically producing imidazol-1-yl-oxime derivatives having the chemical formulae ($II_t$) and ($II_c$):

($II_t$)

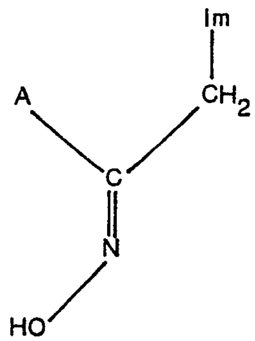

trans

and

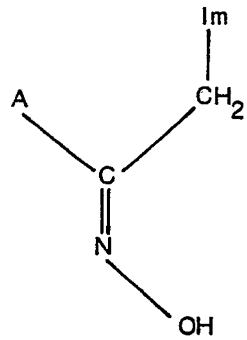

($II_c$)

cis

and imidazol-1-yl-oxime ether derivatives having the chemical formulae ($I_t$) and ($I_c$) and, if need be, oxime ether adducts with teleologically tolerable acids

($I_t$)

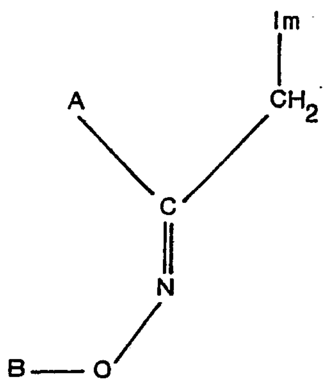

trans

and

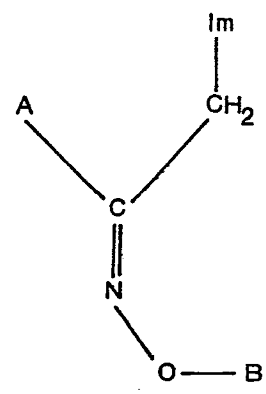

($I_c$)

cis

in which

A is phenyl which is optionally once or twice substituted with halogene,

B is a saturated aliphatic radical having up to 8 carbon atoms, and which can be bound to a phenyl radical as defined above for A, and

Im is the 1-H-imidazol-1-yl-radical,

by reacting a corresponding 1-substituted 2-haloethanone having the formula (IV):

$$A—CO—CH_2—Hal \qquad (IV)$$

in the presence of a solvent with imidazole or with a corresponding imidazole derivative and with hydroxylamine or with a hydroxylamine derivative forming free hydroxylamine under the conditions of reaction, to form an imidazol-1-yl-oxime having the formula (II):

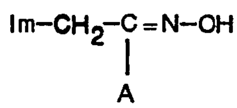

(II)

and, if need be, by a respective etherification of the metal oximate of (II) in a polar solvent with a halogene compound having the formula

$$Hal—B \qquad (III)$$

and, if need be, then precipitating the product in the form of its acid adduct, A, B and Im in the aforementioned formulae having the same meaning as defined above and Hal being halogene, characterized in that, for making the *cis-oxime* ($II_c$) the ethanone of formula (IV) at first is reacted with

imidazole or a corresponding imidazole derivative forming a corresponding 2-(imidazol-1-yl) derivative and then reacting the product in a heated alcoholic solution with hydroxylamine or a hydroxylamine derivative forming free hydroxylamine under the conditions of the reaction, whereas for making the *trans-oxime* ($II_t$) the ethanone of the formula (IV) at first is reacted in an alcoholic solution having a temperature only slightly above room temperature at maximum with hydroxylamine or with a hydroxylamine derivative setting free hydroxylamine to form 2-halogene-cis-oxime having the formula ($V_c$)

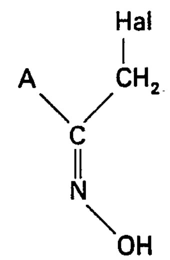

($V_c$)

and reacting said product then with imidazole or a corresponding imidazole derivative in an aprotic solvent at a temperature in a range from 0 to 5°C forming the trans-oxime of formula ($II_t$) via cis-trans transformation, and in that for making the cis- and trans-oxime ethers of formulae ($I_c$) and ($I_t$), respectively, the corresponding stereoisomeric oxime having the formulae ($II_c$) and ($II_t$), respectively, is converted into the corresponding metal oximate at room temperature, and in that the etherification finally is carried out at a reaction temperature of not above 40°C.

2. A method of claim 1, characterized in that, for making the cis-isomers of formula ($I_c$) KOH is used as the metal compound adapted for forming the metal oximate, and for making the trans-isomers of the formula ($I_t$) NaH is used as such metal oximate forming metal compound, whereat in the former case KOH preferably is added in an amount of 5 to 15 mole% less than the equimolar amount based in each case on the molar amount of the oxime derivative of formula ($II_c$).

3. A method according to one of the claims 1 or 2, characterized in that, the etherification is carried out in acetone or dimethylformamide (DMF) as the solvent using preferably acetone as a solvent for making the cis-isomers of formula ($I_c$) and using DMF for making the trans-isomers of formula ($I_t$).

4. A method according to the claims 1, 2 and 3 for producing (Z)-1-(2,4-dichlorophenyl)-2-(1H-imidazol-1-yl)-O-(2,4-dichlorobenzyl)-ethanone oxime (cis-isomer) having the formula

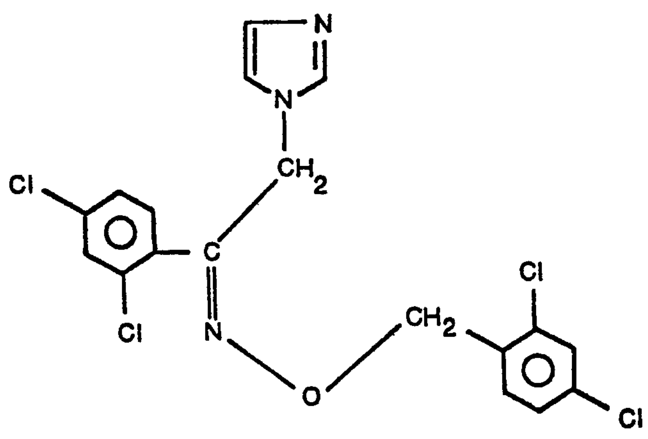

and the nitrate thereof having the formula

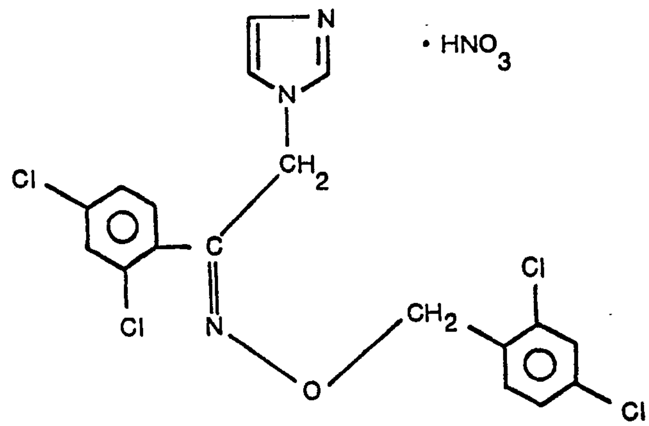

respectively, characterized in that, (Z)-1-(2,4-dichlorophenyl)-2-(1H-imidazol-1-yl)-ethanone oxime is suspended in about the five-fold amount of acetone based on the weight of said oxime and is added with finely pulverized KOH in an amount not surpassing the equimolar amount, preferably in an amount of up to 15 mole% less than the equimolar amount while vigorously stirring until all of the oxime is completely dissolved, in that the solution after about 1h is added in one single portion with about the equimolar amount of 2,4-dichlorobenzyl chloride and then stirring for about further 4h while maintaining the reaction mixture at a temperature of not above 40°C while additionally cooling, if need be, and in that the reaction mixture then is poured into water recovering the oxime ether as usual, or, in that the aqueous reaction mixture is added with aqueous 2n $HNO_3$ for precipitating and separating the nitrate and separating the precipitated nitrate by filtration and in that the nitrate is recristallized from ethanole, if need be.

5. A method of claim 1, characterized in that, hydroxylammonium chloride is used as hydroxylamine forming reagent in the oximation reactions.

6. A method according to one of the claims 1 or 5, characterized in that, the oximation of said 2-imidazol-1-yl derivatives for forming the cis-oxime of formula ($II_c$) is carried out in an alcanole having 1 or 2 carbon atoms, especially in methanol, preferably in the presence of an alcalimetalhydroxyde at reflux temperature.

7. A method according to one of the claims 1 or 5, characterized in that, the oximation of said 2-haloethanone of formula (IV) for forming said 2-halo-cis-oxime of formula ($V_c$) is carried out in methanol solvent at room temperature.

8. A method according to one of the claims 1, 5 or 7, characterized in that, the imidazolification of said 2-halo-cis-oxime of formula ($V_c$) taking place under cis-trans transformation is carried out in chloroform.

9. A method of claim 1 for producing (Z)-1-(2,4-dichlorophenyl)-2-(1H-imidazol-1-yl)-O-(2,4-dichlorobenzyl)-ethanone oxime and the acid adducts thereof with teleologically acceptable acids, characterized in that, (Z)-1-2,4-dichlorophenyl)-2-(1H-imidazol-1-yl)-ethanone oxime and 2,4-dichlorobenzyl chloride are used as the starting compounds and in that the thus obtained ether then is precipitated in the form of its acid adduct, if need be.